(19) Europäisches Patentamt
European Patent Office
Office européen des brevets

(11) EP 1 386 600 A1

(12) **EUROPEAN PATENT APPLICATION**

(43) Date of publication:
**04.02.2004 Bulletin 2004/06**

(51) Int Cl.[7]: **A61K 7/027**

(21) Application number: **03291845.0**

(22) Date of filing: **25.07.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **02.08.2002 FR 0209887**
**02.08.2002 FR 0209889**
**02.08.2002 FR 0209888**

(71) Applicant: **L'OREAL**
**75008 Paris (FR)**

(72) Inventor: **Tournilhac, Florence**
**75011 Paris (FR)**

(74) Representative: **Boulard, Denis**
**L'OREAL - D.I.P.I.**
**25-29 Quai Aulagnier**
**92600 Asnières (FR)**

(54) **Composition gelled with a dextrin ester**

(57) The invention relates to a physiologically acceptable composition, especially a cosmetic composition, containing a continuous liquid fatty phase, which is gelled or thickened with at least one dextrin ester with a degree of substitution of less than 2. The dextrin ester makes it possible to obtain a stick that does not exude, and which gives a glossy, non-migrating and long-lasting deposit over time when applied.



Printed by Jouve, 75001 PARIS (FR)

## Description

The present invention relates to a cosmetic or pharmaceutical composition that may be used for caring for and/or making up the skin, semi-mucous membranes, mucous membranes and/or the integuments.

It is common practice to find a thickened fatty phase in cosmetic or dermatological products; this is especially the case in ointments, anhydrous exfoliant or care gels, and solid compositions, for instance deodorants, balms and lipsticks.

It is moreover known practice to thicken oils with thickening polymers. Unfortunately, the known oil thickeners must be used in large amounts in order to obtain a gel of high viscosity. However, an excessive amount of thickener gives the composition insufficient properties when it is intended for cosmetics, especially a sticky feel and a lack of slipperiness, these drawbacks possibly being very inconvenient, or even unacceptable.

Styrene/ethylene-propylene/styrene triblock polymers such as those described in document US 5 221 534 used in large amount to thicken the oily phase of a foundation have poor cosmetic properties: the makeup is very sticky and thick during application, and gives the user a sensation of heaviness. In addition, these polymers do not make it possible to obtain a foundation that is stable for two months at 45°C: the product releases oil at the surface of the composition and is therefore no longer uniform. The user must then shake the composition well before using it. If the composition is not shaken or is poorly shaken, the application of this composition to the skin leaves an uncomfortable greasy sensation and the makeup obtained is not uniform, traces of colour being visible on the skin. Moreover, an excessively thick foundation is difficult to render satisfactorily uniform before the user applies it to the skin, thus making it difficult to obtain a uniform makeup result on the skin.

The present invention is directed towards providing compositions containing a continuous or external liquid fatty phase thickened or gelled with an agent of the dextrin ester type.

Dextrin esters are generally known as emulsion stabilizers and as oil thickeners in the prior art.

In particular, patent US 4 780 145 describes an anhydrous composition containing a cyclic polydimethylsiloxane, a dextrin ester with a fatty acid and petroleum jelly or a plant oil. The said document illustrates the ability of dextrin esters to gel both polar and a polar oils.

Similarly, patent application JP 63 216 817 describes a gel containing a cyclic silicone of low molecular weight, a fatty phase, i.e. petroleum jelly or lanolin, and a dextrin ester.

Patent application JP 04 149 116 describes a transparent composition containing a dextrin ester - such that its degree of polymerization is between 3 and 100 and the degree of substitution is between 1.4 and 2.4 - and liquid paraffin.

Patent application WO 97/11678 describes an antiperspirant gel gelled especially with a dextrin ester. The dextrin ester described has a degree of esterification of greater than 2. This composition, which is presented as being stable, has the drawback of exuding.

Patent application FR 0 008 157 relates to aqueous emulsions stabilized with a combination of a dextrin ester and a hydrophilic gelling agent.

Patent US 5 840 883 describes dextrin esters with a degree of substitution of between 1 and 3 and preferably between 1.2 and 2.8, which have improved gelling properties.

The Applicant has observed that the compositions of the prior art have a tendency to become covered with droplets of fatty substance over time, and do so all the more quickly the higher the temperature and/or humidity conditions are. This phenomenon of exudation of fatty substances is very detrimental, since it puts off users.

One aim of the present invention is thus to provide a composition thickened or gelled with a dextrin ester that does not exude and/or that does not release oil at the surface of the composition and/or that is stable. An aim of the present invention is also to provide a composition of uniform colour that has all these advantages. An other aim of the present invention is to provide a composition that gives a glossy deposit on the skin or the lips. An other aim of the present invention is to provide a composition that gives a comfortable film deposited on the keratinic materials.

The Applicant has found, surprisingly, that the use of a particular dextrin ester makes it possible to thicken or even to gel certain liquid fatty phases without observing any exudation and/or any release of oil at the surface of the composition. When the composition obtained is applied to the lips, it can gives a glossy, non-migrating, comfortable, long-lasting film of uniform colour that does not exude.

Specifically, the Applicant has found that the use, in the composition, of a dextrin ester with a degree of esterification of less than 2 and which shows affinity for the oil or the mixture of oils of the liquid fatty phase, makes it possible to avoid any exudation and to obtain a composition that has all these advantages.

One subject of the present invention is thus compositions containing a continuous liquid fatty phase, thickened or gelled with an agent of the dextrin ester type with a degree of substitution of less than 2. This dextrin ester has the advantage of retaining the oils contained in the fatty phase without making the composition matt, unlike waxes. It also makes it possible to obtain a composition that does not exude.

The Applicant has also found, surprisingly, that the use of a particular dextrin ester makes it possible to thicken

or even to gel anhydrous compositions, which do not exude, without the need to use waxes. When the composition obtained is applied to the lips, it can give a glossy, non-migrating, comfortable film of uniform colour that does not exude.

Specifically, the Applicant has found that the use, in a wax-free anhydrous composition, of a dextrin ester with a degree of esterification of less than 2, makes it possible to avoid any exudation and to obtain a composition that has all these advantages.

This dextrin ester has the advantage of retaining the oils contained in the fatty phase without making the composition matt, unlike waxes. It also makes it possible to obtain a composition of improved stability.

The Applicant has also found, surprisingly, that the use of a mixture of a dextrin ester with a degree of esterification of less than 2, and of a dextrin ester with a degree of substitution of greater than 2 makes it possible to obtain thickened or gelled compositions that are stable over time. Furthermore, it is also possible to obtain transparent or translucent composition. When the composition obtained is applied to the lips, it can gives a glossy, non-migrating, comfortable film of uniform colour.

This mixture of dextrin esters has the advantage of retaining the oils contained in the fatty phase with making it possible to obtain a composition with improved stability and/or with improved shiny.

The invention applies not only to makeup products for the lips, but also to care and/or treatment products for the skin, including the scalp, and the lips, for instance antisun products, especially in stick form, for facial skin, skin makeup products, for both the human face and body, for instance foundations, especially foundations cast in stick or in dish form, concealer products, eye-shadows and transfer tattoo products, body hygiene products, for instance deodorants, especially in stick form, shampoos and conditioners, and eye makeup products, for instance eyeliners, in particular in pencil form and mascaras, more especially in the form of cakes, and also body and facial care products.

For the purposes of the invention, the expression "dextrin" means a mixture of glucose, malhose and higher molecular weight sacharides formed by acid hydrolysis of starch. It is usually marketed as corn syrup. In one embodiment, dextrin.

For the purposes of the invention, the expression "liquid fatty phase" means a fatty phase that is liquid at room temperature (25°C) and atmospheric pressure (760 mmHg), composed of one or more fatty substances that are liquid at room temperature, also known as "oils", which are generally mutually compatible.

The expression "anhydrous cosmetic composition" means a composition to which water is not added during the formulation, comprising a fatty outer or continuous phase that represents up to 98% by weight of the composition and preferably up to 99.5% by weight.

For the purposes of the invention, the term "thickened" means a composition whose viscosity is increased by adding the dextrin ester, and which flows under its own weight over time.

For the purposes of the invention, the term "gelled" means a composition whose viscosity is increased by adding the dextrin ester, and which does not flow under its own weight over time.

For the purposes of the invention, the expression "transparent" or "translucent" means that a portion of visible light gets through a film of the composition according to the invention, having a particular thickness.

When said portion of visible light diffuses, the composition is translucent and when said portion of visible light does not diffuse, the composition is transparent.

The translucent or transparent feature of the composition is determined as follows : the composition is eventually heated to be poured into a 30 ml Volga pot. The composition is made cold to ambient temperature for 24 hours.

A black cross with 2 nm thick lines is obtain on a white sheet of paper, and the pot filled with the composition is placed on top of the paper sheet.

If the cross can be seen at maked eye under daylight at a 40 cm maximum observation distance, then the composition is translucent or transparent.

A first subject of the present invention is a composition containing a continuous liquid fatty phase gelled or thickened with a sufficient amount of at least one fatty acid ester of dextrin with a degree of substitution of less than 2 on the basis of one repeating unit, for example one glucose unit,, the said fatty phase containing an oil or a mixture of oils that has solubility parameters δd, δp and δh satisfying the following conditions :

$$[4(\delta d-\delta d_e)^2 + (\delta p-\delta p_e)^2 + (\delta h-\delta h_e)^2]^{1/2} \leq 10$$

$\delta d_e$, $\delta p_e$ and $\delta h_e$ being the solubility parameters of the dextrin ester, the liquid fatty phase and the dextrin ester forming a physiologically acceptable medium.

A second subject of the present invention is thus a wax-free anhydrous composition containing a continuous liquid fatty phase thickened or gelled with a sufficient amount of at least one fatty acid ester of dextrin with a degree of substitution of less than 2 on the basis of one repeating unit, for example one glucose unit,, the liquid fatty phase and the dextrin ester forming a physiologically acceptable medium.

A third subject of the present invention is a composition containing a continuous liquid fatty phase gelled or

thickened with a sufficient amount of a mixture containing at least one fatty acid ester of dextrin with a degree of substitution of less than 2 on the basis of one repeating unit, for example one glucose unit, and at least one fatty acid ester of dextrin with a degree of substitution of greater than 2 on the basis of one repeating unit, for example one glucose unit,, the liquid fatty phase and the dextrin esters forming a physiologically acceptable medium.

***Dextrin ester***

According to any one embodiment, the fatty acid ester of dextrin with a degree of substitution of less than 2 on the basis of one repeating unit, for example one glucose unit, corresponds to formula (I):

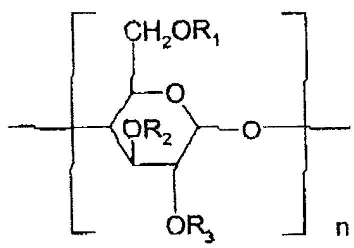

(I)

in which:

- the radicals $R_1$, $R_2$ and $R_3$, which may be identical or different, are chosen from hydrogen or an acyl group (R-CO-) in which the radical R is a linear or branched, saturated or unsaturated hydrocarbon-based group containing 6 to 50, especially 8 to 30, or even 12 to 22 and better still 12 to 18 carbon atoms, with the proviso that at least one of the said radicals $R_1$, $R_2$ or $R_3$ is other than hydrogen,
- n is an integer between 3 and 150, especially 10 and 100, and preferably 15-40.

According to another embodiment, the fatty acid ester of dextrin with a degree of substitution of less than 2 on the basis of one repeating unit, for example one glucose unit, corresponds to formula (II):

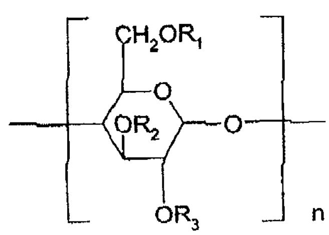

(II)

in which:

- the radicals $R_1$, $R_2$ and $R_3$ represent hydrogen or an acyl group (R-CO-) in which the radical R is a linear or branched, saturated or unsaturated hydrocarbon-based group containing 6 to 50, especially 8 to 30, or even 12 to 22 and better still 12 to 18 carbon atoms, with the proviso that at least one of the said radicals $R_1$, $R_2$ or $R_3$ is other than hydrogen,
- n is an integer between 3 and 150, especially 10 and 100, and preferably 15-40.

The radical R-CO- of the dextrin ester of formula (I) or (II) is chosen from caprylyl, caproyl, lauroyl, myristyl, palmityl, stearyl, eicosanyl, docosanoyl, isovaleryl, 2-ethylbutyryl, ethylmethylacetyl, isoheptanyl, 2-ethylhexanyl, isononanyl, isodecanyl, isotridecanyl, isomyristyl, isopalmityl, isostearyl, isohexanyl, decenyl, dodecenyl, tetradecenyl, myristyl, hexadecenoyl, palmitoleyl, oleyl, elaidyl, eicosenyl, sorbyl, linoleyl, linolenyl, punicyl, arachidonyl and stearoyl radicals, and mixtures thereof.

The radical R-CO is advantageously linear. R-CO is preferably the palmitic radical.

Preferably, a dextrin palmitate is used, such that the degree of substitution is less than 1.9, preferably less than 1.8 and more preferably between 1.5 and 1.7.

n advantageously ranges from 25 to 35, preferably from 27 to 33 and better still is equal to 30.

The weight-average molecular weight of the dextrin ester with a degree of substitution of less than 2 is preferably between 10 000 and 30 000 and more preferably between 15 000 and 20 000. The weight-average molecular weight is determined by gas chromatography, with a polystyrene standard.

Some of these dextrin esters are commercially available, especially under the name Rheopearl TL from the company Chiba Flour.

The fatty acid ester of dextrin with a degree of substitution of less than 2 on the basis of one repeating unit, for example one glucose unit, may be present in the composition according to the invention in an amount ranging from 1% to 50%, preferably from 4 % to 30 %, preferably from 4% to 25%, preferably from 5% to 25%, preferably from 4 % to 25 % and preferably from 10% to 25% by weight, relative to the total weight of the composition.

The fatty acid ester of dextrin with a degree of substitution of greater than 2 on the basis of one repeating unit, for example one glucose unit, may be present in the composition according to the invention in an amount ranging from 1% to 50%, preferably from 4 % to 30 %, preferably from 4% to 25%, preferably from 5% to 25%, preferably from 4 % to 25 % and preferably from 10% to 25% by weight, relative to the total weight of the composition.

The thickening or gelation of the oils of the liquid fatty phase, which may be partly modified by the nature and amount of the dextrin ester used, is such that a rigid structure is obtained, in the form of a tube or a stick, or in pasty form. The tubes, when they are coloured, make it possible after application to obtain a glossy deposit that is uniform in colour and that does not migrate in the wrinkles and fine lines of the skin in particular surrounding the lips, but also the eyes.

According to the invention, the thickening or gelation of the liquid fatty phase is obtained with the aid of one or more dextrin esters with a degree of substitution of less than 2 on the basis of one repeating unit, for example one glucose unit, in particular one or more dextrin esters of formula (I) or (II).

According to any one embodiment, the fatty acid ester of dextrin with a degree of substitution of greater than 2 on the basis of one repeating unit, for example one glucose unit, corresponds to formula (III):

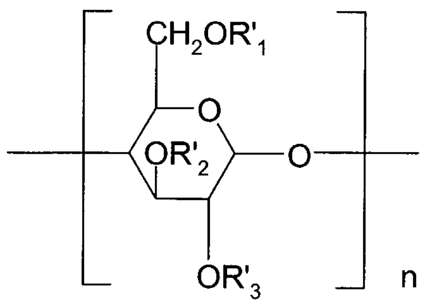

(III)

in which:

- the radicals R'1, R'2 and R'3, which may be identical or different, are chosen from hydrogen or a acyl group (R'-CO-) in which the radical R' is a linear or branched, saturated or unsaturated hydrocarbon-based group containing 6 to 50, especially 8 to 30, or even 12 - 22 and better still 12 - 18 carbon atoms, with the proviso that at least one of the said radicals R'1, R'2 or R'3 is other than hydrogen,
- n is an integer between 3 and 150, especially between 10 and 100 and preferably 15 - 40;
  R' and n may have the same meaning of R and n described previously.

Preferably, a fatty acid ester of dextrin with a degree of substitution of greater than 2 on the basis of one repeating unit, for example one glucose unit, is used such that the degree of substitution is greater than 2.1 and preferably between 2.1 and 2.3.

The weight-average molecular weight of the fatty acid ester of dextrin with a degree of substitution of greater than 2 on the basis of one repeating unit, for example one glucose unit, is preferably between 10 000 and 30 000 and more preferably between 15 000 and 20 000. The weight-average molecular weight is determined by gas chromatography with a polystyrene standard.

Examples of dextrin esters of formula (III) according to the invention that may be mentioned include Rheopearl KL sold by the company Chiba Flour.

The mixture of dextrin esters may be present in the composition according to the invention in an amount of less than or equal to 50%, particularly from 5 % to 50 %, preferably from 5% to 40% and preferentially from 5% to 25% by weight, and most preferably from 9 % to 25 %, relative to the total weight of the composition.

The fatty acid ester of dextrin with a degree of substitution of less than 2 and the fatty acid ester of dextrin with a degree of substitution of greater than 2 are preferably in relative mass proportions ranging from 1/10 to 3/1, preferentially from 1/8 to 3/2 and more preferentially from 1/2 to 3/2, preferably from 0.8 to 1.2, preferably from 0.9 to 1.1, and particularly from 1/2 to 1/1.

***Fatty phase***

According to the three subjects of the invention, the liquid fatty phase of the composition according to the invention may contain a volatile liquid organic phase.

According to the second and the third subjects of the invention, the liquid fatty phase of the composition according to the invention may also contain a non-volatile liquid organic phase.

According to the first subject of the invention, the liquid fatty phase of the composition may also contain a non-volatile liquid organic oil different from the oil showing specific solubility parameters.

The expression "volatile organic phase" means any non-aqueous medium capable of evaporating from the skin in less than one hour at room temperature and atmospheric pressure. This volatile phase especially comprises oils with a vapour pressure, at room temperature (25°C) and atmospheric pressure (760 mm Hg), ranging from 0.13 Pa to 40 000 Pa ($10^{-3}$ to 300 mm Hg), preferably ranging from 1.3 Pa to 13 000 Pa (0.1 to 100 mm Hg) and preferentially ranging from 1.3 Pa to 13 000 Pa (0.01 to 10 mm Hg).

The expression "non-volatile organic phase" means any medium capable of remaining on the skin for several hours. A non-volatile liquid organic phase in particular has a non-zero vapour pressure at room temperature and atmospheric pressure, of less than 0.001 mm Hg (0.13 Pa).

The liquid fatty phase may comprise at least one oil chosen especially from carbon-based oils, hydrocarbon-based oils, fluoro oils and/or silicone oils of mineral, animal, plant or synthetic origin, alone or as a mixture, provided that they form a uniform and macroscopically stable mixture and that they are suitable for the intended use.

The term "hydrocarbon-based oil" means an oil formed essentially from, or consisting of, carbon and hydrogen atoms, and optionally oxygen and nitrogen atoms, and containing no silicone or fluorine atoms. It may contain alcohol, ester, ether, carboxylic acid, amine and/or amide groups.

The total liquid fatty phase of the composition (i.e. the total amount of oil(s)) can represent from 5% to 95% by weight, preferably from 20% to 75% by weight and preferentially from 30% to 70% by weight, relative to the total weight of the composition.

Advantageously, the liquid fatty phase may contain one or more organic oils that are volatile at room temperature, for instance volatile cosmetic oils. These oils are favourable for obtaining a transfer-resistant deposit with good staying power. These volatile oils also facilitate the application of the composition to the skin. They may be hydrocarbon-based oils, silicone oils and/or fluoro oils and may optionally comprise alkyl or alkoxy groups, pendent or at the end of silicone chain.

As volatile organic oils that may be used in the invention, mention may be made of:

- linear or cyclic silicone oils with a viscosity at room temperature of less than 8 $mm^2/s$ and especially containing from 2 to 7 silicone atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane;
- hydrocarbon-based volatile oils containing from 8 to 16 carbon atoms and mixtures thereof, and especially $C_8$-$C_{16}$ branched alkanes, for instance $C_8$-$C_{16}$ isoalkanes (also known as isoparaffins), isododecane, isodecane, isohexadecane and, for example, the oils sold under the trade names "Isopar" or "Permethyl", branched $C_8$-$C_{16}$ esters, for instance isohexyl neopentanoate, and mixtures thereof; isododecane is preferably used;
- and mixtures thereof.

Advantageously, the volatile organic oil(s) represent(s) from 0.1% to 80%, preferably from 1% to 60% and better still from 5% to 50% of the total weight of the composition.

The composition may also comprise a non-volatile oil.

As non-volatile oils that may be used in the invention, mention may be made of hydrocarbon-based oils of mineral or synthetic origin, such as linear or branched hydrocarbons, for instance liquid paraffin or its derivatives, liquid petroleum jelly, polydecenes, hydrogenated polyisobutene such as Parleam sold by the company Nippon Oil Fats, and squalane of synthetic or plant origin; oils of animal origin, for instance mink oil, turtle oil, perhydrosqualene; hydrocarbon-based oils of plant origin with a high triglyceride content, consisting of fatty acid esters of glycerol, the fatty acids of which may have varied chain lengths, these chains possibly being linear or branched, and saturated or unsaturated,

especially fatty acid triglycerides especially of 4 to 22 carbon atoms, for instance the triglycerides of heptanoic and octanoic acid, and of capric/caprylic acid, or alternatively hydroxylated triglycerides, such as sweet almond oil, beauty-leaf oil, palm oil, grapeseed oil, sesame seed oil, arara oil, rapeseed oil, sunflower oil, cotton seed oil, apricot oil, castor oil, alfafa oil, marrow oil, blackcurrant oil, macadamia oil, musk rose oil, hazelnut oil, avocado oil, jojoba oil, olive oil, cereal (maize, wheat, barley or rye) germ oil and karite butter; fatty acid esters, in particular of 4 to 22 carbon atoms, and especially of octanoic acid, of heptanoic acid, of lanolic acid, of oleic acid, of lauric acid or of stearic acid, for instance propylene glycol dioctanoate, propylene glycol monoisostearate, polyglyceryl-2 diisostearate or neopentyl glycol diheptanoate; synthetic esters of formula $R_1COOR_2$ in which $R_1$ represents a linear or branched higher fatty acid residue containing from 7 to 40 carbon atoms and $R_2$ represents a branched hydrocarbon-based chain containing from 3 to 40 carbon atoms, for instance purcellin oil (cetostearyl octanoate), isononyl isononanoate, $C_{12}$ to $C_{15}$ alkyl benzoate, 2-ethylhexyl palmitate, 2-octyldodecyl stearate, 2-octyldodecyl erucate, isostearyl isostearate, 2-octyldodecyl benzoate, alcohol or polyalcohol octanoates, decanoates or ricinoleates, isopropyl myristate, isopropyl palmitate, butyl stearate, hexyl laurate, diisopropyl adipate, 2-ethylhexyl palmitate, 2-hexyldecyl laurate, 2-octyldecyl palmitate, 2-octyldodecyl myristate, 2-diethylhexyl succinate, diisostearyl malate or isodecyl neopentanoate; hydroxylated esters, for instance isostearyl lactate, octyl hydroxystearate, octyldodecyl hydroxystearate, diisostearyl malate, triisocetyl citrate, glyceryl triisostearate or diglyceryl triisostearate; diethylene glycol diisononanoate; pentaerythritol esters; esters of aromatic acids and of alcohols containing 4 to 22 carbon atoms, especially tridecyl trimellitate; $C_8$-$C_{26}$ higher fatty acids such as oleic acid, linoleic acid, linolenic acid or isostearic acid; $C_8$-$C_{26}$ higher fatty alcohols such as oleyl alcohol, linoleyl alcohol, linolenyl alcohol, isostearyl alcohol or octyldodecanol; synthetic esters containing at least 7 carbon atoms, silicone oils such as linear polydimethylsiloxanes (PDMS) that are liquid at room temperature, and optionally phenylated, such as phenyltrimethicones, phenyltrimethylsiloxydiphenylsiloxanes, diphenyl dimethicones, diphenyl methyldiphenyl trisiloxanes, 2-phenylethyl trimethylsiloxysilicates that are liquid, optionally substituted with aliphatic and/or aromatic groups, for instance alkyl, alkoxy or phenyl groups, pendent and/or at the end of a silicone chain, these groups containing from 2 to 24 carbon atoms and being optionally fluorinated, or with functional groups such as hydroxyl, thiol and/or amine groups; polysiloxanes modified with fatty acids, with fatty alcohols or with polyoxyalkylenes, for instance dimethicone copolyols or alkylmethicone copolyols; liquid fluorosilicones; and mixtures thereof.

The composition may comprise one or more non-volatile oils in a content ranging from 0.1% to 80%, preferably ranging from 1% to 60% by weight and preferentially ranging from 5% to 50% by weight, relative to the total weight of the composition.

According to the first subject of the present invention, the composition contains a continuous liquid fatty phase gelled or thickened with a sufficient amount of at least one fatty acid ester of dextrin with a degree of substitution of less than 2 on the basis of one repeating unit, for example one glucose unit, the said fatty phase containing an oil or a mixture of oils that has solubility parameters δd, δp and δh satisfying the following conditions :

$$[4(\delta d-\delta d_e)^2 + (\delta p-\delta p_e)^2 + (\delta h-\delta h_e)^2]^{1/2} \leq 10$$

$\delta d_e$, $\delta p_e$ and $\delta h_e$ being the solubility parameters of the dextrin ester, the liquid fatty phase and the dextrin ester forming a physiologically acceptable medium. The definition and calculation of the solubility parameters in the three-dimensional Hansen solubility space are described in the article by C.M. Hansen: "The three dimensional solubility parameters" J. Paint Technol. 39, 105 (1967).

The three Hansen solubility parameters - δd, δp and δh - characterize, for a given constituent, the energies corresponding, respectively, to the dispersive interactions, polar interactions and interactions of hydrogen bonding type existing between the molecules of this constituent.

In order for the oil or the mixture of oils of the liquid fatty phase to be compatible with the dextrin ester used, the solubility parameters of the said oil or of the said mixture of oils are chosen so as to be within the solubility sphere of the said dextrin ester, defined by a centre of coordinates $\delta d_e$, $\delta p_e$ and $\delta h_e$ and a radius R of not more than 10. The radius is calculated according to the following formula:

$$R = [4(\delta d-\delta d_e)^2 + (\delta p-\delta p_e)^2 + (\delta h-\delta h_e)^2]^{1/2}.$$

According to any one embodiment, R is less than or equal to 8 and preferably to 5.

The values of the Hansen solubility parameters for the dextrin ester are especially such that $17 \leq \delta d_e \leq 19.1 \leq \delta p_e \leq 2$ and $9 \leq \delta h_e \leq 11$.

The values of the Hansen solubility parameters for Rheopearl TL are, respectively,

$\delta d_e = 18.2$, $\delta p_e = 1.5$ and $\delta h_e = 9.8$.

The liquid fatty phase may comprise a mixture of oils. In this case, the solubility parameters of the mixture are determined from those of the oils taken separately, according to the following relationships: $\delta_{Dmixt} = \sum_i xi\ \delta_{Dl}$ $\delta_{pmixt} = \sum_i xi\ \delta_{pi}$ and $\delta_{hmixt} = \sum_i xi\ \delta_{hi}$ in which xi represents the volume fraction of the oil i in the mixture.

As oils which, alone, satisfy the conditions of the present invention, mention may be made of

- fatty acid triglycerides, especially of 4 to 22 carbon atoms, for instance heptanoic, octanoic and capric/caprylic acid triglycerides,
- hydroxylated triglycerides, for instance castor oil,
- synthetic alcohols containing from 12 to 26 carbon atoms, for instance octyldodecanol, 2-butyloctanol, 2-hexyldecanol and 2-undecylpentadecanol,
- fatty acid esters, especially of 4 to 22 carbon atoms, for instance propylene glycol dioctanoate, propylene glycol monoisostearate, neopentyl glycol diheptanoate, diethylene glycol diisononanoate; polyglyceryl 2 diisostearate, oleyl alcohol,
- hydroxylated synthetic esters, for instance isostearyl lactate, octyl hydroxystearate, octyldodecyl hydroxystearate, diisostearyl malate or triisocetyl citrate,
- esters of aromatic acids and of alcohols containing 4 to 22 carbon atoms, especially tridecyl trimellitate,
- or mixtures thereof.

Table 1 below gives the value of the radius of the sphere $R = [4(\delta d-18.2)^2+(\delta p-1.5)^2+(\delta h-9.8)^2]^{1/2}$ for various oils relative to the values of the Hansen solubility parameters for Rheopearl TL. The following oils fall within the context of the present invention, without limiting the scope thereof. Other oils that satisfy the conditions of the invention are available.

TABLE 1

| Name of the oil | δd | δp | δh | Distance between the oil and Rheopearl TL |
|---|---|---|---|---|
| Castor oil | 16.79 | 1.27 | 9 | 2.9 |
| 2-Octyldodecanol | 16.36 | 1.43 | 7.56 | 4.3 |
| Oleyl alcohol | 16.28 | 1.6 | 8.01 | 4.2 |
| Caprylic/capric acid triglyceride | 16.64 | 1.69 | 6.47 | 4.6 |
| 2-Ethylhexyl glyceryl ether palmitate | 16.53 | 1.71 | 7.98 | 3.8 |
| Hexyldecanol | 16.26 | 1.75 | 8.37 | 4.1 |
| 2-Ethylhexyl glyceryl behenate | 16.57 | 1.42 | 7.27 | 4.1 |
| Propylene glycol monoisostearate | 16.36 | 1.89 | 8.53 | 3.9 |
| Pentaerythrityl tetraethyl-2-hexanoate | 16.55 | 1.53 | 6.62 | 4.6 |
| Polyglyceryl 3 diisostearate | 16.96 | 1.64 | 10.27 | 2.5 |
| Isostearyl alcohol | 16.32 | 1.58 | 7.94 | 4.2 |
| Phytanetriol | 16.5 | 2.52 | 13.2 | 4.9 |
| Triisocetyl citrate | 16.77 | 1.09 | 6.74 | 4.2 |
| Triisoarachidyl citrate | 16.77 | 0.9 | 6.12 | 4.7 |
| Diisostearyl malate | 16.61 | 1.26 | 7.08 | 4.2 |
| Triisostearyl citrate | 16.77 | 0.99 | 6.4 | 4.5 |
| Oxyethylene (7 OE) glyceryl triacetate | 16.97 | 2.98 | 9.61 | 2.9 |
| Tridecyl trimellitate | 17.49 | 1.12 | 5.23 | 4.8 |
| Glyceryl triheptanoate | 16.62 | 1.99 | 7.01 | 4.2 |
| Polyglyceryl-2 triisostearate | 16.7 | 1.06 | 6.61 | 4.4 |
| Undecylpentadecanol | 16.45 | 1.12 | 6.69 | 4.7 |
| PPG10 butanediol | 16.77 | 2.38 | 10.73 | 3.1 |
| Glyceryl triacetate | 16.42 | 4.57 | 10.63 | 4.8 |
| Octyl hydroxystearate | 16.43 | 1.55 | 7.73 | 4.1 |

TABLE 1 (continued)

| Name of the oil | δd | δp | δh | Distance between the oil and Rheopearl TL |
|---|---|---|---|---|
| C12-13 alkyl lactate | 16.25 | 2.47 | 9.76 | 4.0 |
| Isostearyl lactate | 16.36 | 1.89 | 8.53 | 3.9 |
| 2-Octyldodecyl hydroxystearate | 16.53 | 1.09 | 6.47 | 4.7 |
| Butyl isostearate | 16.6 | 1.21 | 6.72 | 4.5 |
| Pentaerythrityl tetraisononanoate | 16.39 | 1.4 | 6.32 | 5.0 |
| Dipropylene glycol dibenzoate | 18.77 | 2.57 | 7.12 | 3.1 |
| Pentaerythrityl tetrapelargonate | 16.82 | 1.4 | 6.32 | 4.4 |
| Polyglyceryl-2 isostearate | 17.03 | 2.59 | 12.99 | 4.1 |
| Polyglyceryl-2 diisostearate | 16.79 | 1.5 | 8.95 | 2.9 |
| Glyceryl diisostearate | 16.61 | 1.29 | 7.16 | 4.1 |
| Triisodecyl trimellitate | 17.43 | 1.37 | 5.8 | 4.3 |
| Tris(2-ethylhexyl) trimellitate | 17.56 | 1.62 | 6.31 | 3.7 |
| Isofol-12 trimellitate | 17.34 | 1.19 | 5.4 | 4.7 |
| Glyceryl trioctanoate | 16.35 | 1.78 | 6.64 | 4.9 |
| 2-Butyloctanol | 16.12 | 2.26 | 9.52 | 4.2 |

Mixtures of the oils described above may be made with oils that are not found alone in the solubility sphere of the dextrin ester as defined above, provided that the mixture is homogeneous and that the solubility parameters of the mixture of oils satisfy the relationship of the invention. It is within the scope of a person skilled in the art to determine the amounts of each oil to obtain a mixture of oils that satisfies the conditions of the invention.

As oils that do not satisfy the above Hansen relationships, taken individually, mention may be made of:

- volatile or non-volatile silicones,
- oils of mineral origin, for instance liquid petroleum jelly and liquid paraffin,
- oils of plant origin, for instance jojoba oil, sesame oil, rapeseed oil and karite butter,
- synthetic oils, for instance Purcellin oil, 2-ethylhexyl palmitate, 2-octyldodecyl stearate, 2-octyldodecyl erucate, isostearyl isostearate, 2-octyldodecyl benzoate and hydrogenated isoparaffin (6.8 mol of isobutylene), synthetic oils or esters of formula $R_5COOR_6$ in which $R_5$ represents a linear or branched higher fatty acid residue containing from 1 to 40 and better still from 7 to 19 carbon atoms and $R_6$ represents a branched hydrocarbon-based chain containing from 1 to 40 and better still from 3 to 20 carbon atoms, with $R_5 + R_6 \geq 10$, such as, for example, Purcellin oil (cetostearyl octanoate), isononyl isononanoate, $C_{12}$ to $C_{15}$ alkyl benzoate, isopropyl myristate, 2-ethylhexyl palmitate, and octanoates, decanoates or ricinoleates of alcohols or of polyalcohols.

The liquid fatty phase preferably represents from 5% to 95% by weight and preferably from 20% to 75%, and most preferably from 30 % to 70 % relative to the total weight of the composition.

The liquid fatty phase may also contain other oils that are entirely incompatible with the oils described above, i.e. oils that do not form a mixture that is homogeneous to the eye and whose distance in the Hansen space is greater than 10 (in this case there will be no change in the solubility parameters of the initially optimized mixture) chosen especially from silicone oils such as volatile or non-volatile, linear or cyclic polydimethylsiloxanes (PDMS) that are liquid at room temperature; polydimethylsiloxanes comprising alkyl, alkoxy or phenyl groups, pendent and/or at the end of the silicone chain, these groups each containing from 2 to 24 carbon atoms; phenyl silicones, for instance phenyl trimethicones, phenyl dimethicones, phenyl trimethylsiloxy diphenylsiloxanes, diphenyl dimethicones, diphenyl methyldiphenyl trisiloxanes and 2-phenylethyl trimethylsiloxysilicates; volatile or non-volatile, linear or branched fluorocarbons of synthetic or mineral origin; polyglycols.

***Other ingredients***

The composition according to the invention may comprise at least one aqueous phase containing water. The water may be a floral water such as cornflower water and/or a mineral water such as eau de Vittel, eau de Lucas or eau de La Roche Posay and/or a spring water.

The aqueous phase may also comprise organic solvents that are water-miscible (at 25°C), for instance primary

alcohols such as ethanol and isopropanol, glycols such as glycerol, propylene glycol, butylene glycol, dipropylene glycol, diethylene glycol, glycol ethers, $C_1$ to $C_4$ alkyl ethers of monopropylene, dipropylene or tripropylene glycol or of monoethylene, diethylene or triethylene glycol, and mixtures thereof.

The aqueous phase may also comprise stabilizers, for example sodium chloride, magnesium dichloride and magnesium sulphate.

It may also incorporate any water-soluble or water-dispersible compound that is compatible with an aqueous phase, such as gelling agents, film-forming polymers, thickeners and surfactants, and mixtures thereof.

According to one particular embodiment, the aqueous phase, and especially the water, may be present in the composition according to the invention in a content ranging from 1% to 70%, especially from 5% to 50% by weight and in particular from 5% to 20% by weight, relative to the total weight of the composition.

The composition of the invention may also comprise any additional additive usually used in the field under consideration, such as antioxidants, preserving agents, neutralizers, lipophilic gelling agents or liquid non-aqueous compounds, aqueous-phase gelling agents, dispersing agents, and cosmetic active agents. These additives may be present in the composition in a proportion of from 0.0005% to 20% and better still from 0.001% to 10% relative to the total weight of the composition.

As cosmetic active agents that may be used in the invention, mention may be made of vitamins A, E, C, $B_3$ and F, provitamins, for instance D-panthenol, calmant active agents, for instance $\alpha$-bisabolol, aloe vera, allantoin, plant extracts or essential oils, protecting agents or restructuring agents, for instance ceramides, "refreshing" active agents, for instance menthol and its derivatives, emollients (cocoa butter, dimethicone), moisturizers (arginine PCA), anti-wrinkle active agents, essential fatty acids and sunscreens, and mixtures thereof.

Needless to say, a person skilled in the art will take care to select the optional additional additives and/or the amount thereof such that the advantageous properties of the composition according to the invention are not, or are not substantially, adversely affected by the envisaged addition.

The compositions according to the invention have numerous applications and concern all coloured or uncoloured cosmetic products and more particularly lipsticks and foundation.

The composition according to the invention may be in the form of a coloured or uncoloured skincare composition, in the form of an antisun or makeup-removing composition or alternatively in the form of a hygiene composition. If it contains cosmetic active agents, it may then be used as a care base or a non-therapeutic treatment base for the skin such as the hands or the face or for the lips (lip balms, for protecting the lips from cold and/or sunlight and/or wind) or an artificial tanning product for the skin.

The composition of the invention may also be in the form of a coloured makeup product for the skin, in particular for the face, for instance a blusher, a foundation, a makeup rouge or an eye-shadow, a makeup product for the body, for instance a semi-permanent tattoo product, or a makeup product for the lips, for instance a lipstick or a lip gloss, optionally having care properties or non-therapeutic treatment properties, a makeup product for the integuments, for instance a nail varnish, a mascara or an eyeliner, or a hair dye product or haircare product.

Preferably, according to one embodiment of the invention, the composition according to the invention is in the form of a lipstick or a lip gloss. A glossy, non-migrating and long-lasting deposit is produced when this lipstick is applied. According to another embodiment of the invention, the composition is a foundation.

The composition according to the present invention may also contain a pasty fatty substance or a wax.
For the purposes of the invention, the term "pasty compound" means a compound with a melting point ranging from 25 to 60°C and preferably from 30 to 45°C and/or a hardness ranging from 0.001 to 0.5 MPa and preferably from 0.005 to 0.4 MPa.
For the purposes of the present invention, the term "wax" means a lipophilic fatty compound that is solid at room temperature (25°C), with a reversible solid/liquid change of state, having a melting point of greater and 30°C, which may be up to 200°C, a hardness of greater than 0.5 MPa, and having an anisotropic crystal organization in the solid state. By bringing the wax to its melting point, it is possible to make it miscible with the oils and to form a microscopically homogeneous mixture, but on returning the temperature of the mixture to room temperature, recrystallization of the wax in the oils of the mixture is obtained.

In general, the physiologically acceptable medium for the composition according to the invention may comprise, besides the liquid fatty phase, additional compounds that may be chosen from fillers, waxes, oils, gums and/or pasty fatty substances, which are hydrocarbon-based, silicone-based and/or fluorinated, of plant, mineral or synthetic origin, and mixtures thereof.

According to any one embodiment, the physiologically acceptable medium of the composition contains a pasty fatty substance or a wax.

For the purposes of the patent application, the waxes may be hydrocarbon-based waxes, silicone waxes and/or fluoro waxes, optionally comprising ester or hydroxyl functions. They are especially of natural origin, for instance optionally modified beeswax, carnauba wax, candelilla wax, ouricoury wax, Japan wax, cork fibre wax or sugarcane wax, ceresin, paraffin waxes, lignite waxes, microcrystalline waxes, lanolin wax, montan wax, ozokerites, hydrogenated

oils, for instance hydrogenated jojoba oil or products obtained by copolymerization of ethylene, the waxes obtained by Fischer-Tropsch synthesis, fatty acid esters and glycerides that are solid at 45°C, silicone waxes, for instance alkyl or alkoxy poly(di)methylsiloxanes and/or poly(di)methylsiloxane esters that are solid at 45°C, containing from 10 to 45 carbon atoms, and certain fatty acids, for instance stearic acid, myristic acid or behenic acid, and mixtures thereof.

Advantageously, the composition according to the present invention contains not more than 50%, particularly form 0,01 % to 50 %, preferably from 0,1 % to 40 %, and more preferably not more than 30%, and preferably from 0,1 % to 30 % by weight of waxes. According to any one embodiment, the composition is free of waxes. A composition containing 0,5 % or unless than 0,5 % waxes is considered in the present invention as being free of waxes.

The nature and amount of the pasty substances or waxes depend on the desired mechanical properties and textures. As a guide, the wax can represent from 0.01% to 50%, preferably from 0.1 % to 40 %, preferably 2% to 40% and better still from 0.1 % to 30 %, and preferably from 5% to 30% of the total weight of the composition.

According to any one embodiment, the physiologically acceptable medium of the composition contains an additional gelling or thickening system, which results in a transparent or translucent phase.

Transparent waxes such as bis(1,1,1-trimethylolpropane) tetrastearate sold under the name Hest 2T-4S by the company Heterene are chosen, for example.

Moreover, the composition may contain, as additional non-waxy gelling or thickening system. Said system can avantageously results in a transparent phase. Said system is for example :

- N-lauryl-L-glutamate α,γ-di-N-butylamide (LGBA) sold by the company Clariant and described in Zairyo Gijutsu, Vol. 10, No. 2, pp. 48 - 57 (1992)
- monodibenzylidene sorbitol as described in patent US 3 121 332,
- 1,2- and 1,3-cyclohexane derivatives bearing an amide function as described in patent application US 6 410 003.

The expression "non-waxy system" means a gelling agent or thickener not corresponding to the definition of the waxes. A non-waxy system is a lipophilic fatty compound that is solid at room temperature (25°C), which undergoes a reversible solid/liquid change of state, having a melting point of greater than 30°C which may be up to 200°C, a hardness of greater than 0.5 MPa, and having in solid form an anisotropic crystal organization. By bringing a wax to its melting point, it is possible to make it oil-miscible and to form a microscopically homogeneous mixture, but on returning the temperature of the mixture to room temperature, recrystallization of the wax is obtained.

The term "filler" means any colourless particle chosen from lamellar, spherical or oblong mineral or organic fillers that are chemically inert in the composition. According to any one embodiment, the filler is preferably less than 500 nm in size so as to preserve the transparent or translucent nature of the composition.

Mention may be made of talc, mica, silica, kaolin, laponite, polyamide powders, for instance Nylon® powder, poly-β-alanine powder and polyethylene powder, powders of tetrafluoroethylene polymers (Teflon® ), lauroyllysine, starch, boron nitride, particles of acrylic polymer, especially of acrylic acid copolymer, and silicone resin microbeads, precipitated calcium carbonate, dicalcium phosphate, magnesium carbonate, magnesium hydrocarbonate, hydroxyapatite, and mixtures thereof. These fillers may or may not be surface-treated, especially to make them lipophilic.

Preferably, the fillers represent from 0.1% to 60%, preferably from 0.5% to 40% and better still from 1% to 35%, preferably from 1 % to 25 % of the total weight of the first composition, if they are present.

The term "physiologically or cosmetically acceptable" means having a pleasant taste, feel, appearance and/or odour, applicable several days for several months.

The composition according to the invention may be manufactured by the known processes generally used in cosmetics.

The composition may advantageously contain a dyestuff that may be chosen from the lipophilic dyes, hydrophilic dyes, pigments and nacres usually used in cosmetic or dermatological compositions, and mixtures thereof. This dyestuff is generally present in a proportion of from 0.01% to 40%, preferably from 1% to 35% and better still from 5% to 25% relative to the total weight of the composition.

According to one embodiment of the invention, the dyestuff contains dyes and/or pigments and/or nacres so as to obtain a semi-covering or transparent makeup, i.e. a makeup that does not leave the skin, the lips or the integuments exposed. Pigments also make it possible to reduce the sticky feel of the compositions, unlike soluble dyes.

The liposoluble dyes are, for example, Sudan red, DC Red 17, DC Green 6, β-carotene, soybean oil, Sudan brown, DC Yellow 11, DC Violet 2, DC Orange 5 and quinoline yellow. They may represent from 0,1 to 20% and better still from 0.1% to 6% of the weight of the compositions (if present).

The pigments may be white or coloured, mineral and/or organic, and coated or uncoated. Among the mineral pigments that may be mentioned are titanium dioxide, optionally surface-treated, zirconium oxide or cerium oxide, and also iron oxide or chromium oxide, manganese violet, ultramarine blue, chromium hydrate and ferric blue. Among the organic pigments that may be mentioned are carbon black, pigments of D & C type, and lakes based on cochineal carmine or on barium, strontium, calcium or aluminium. The pigments may represent from 0,1 to 40%, preferably from

1 to 35% and better still from 2% to 25% of the total weight of the composition.

The nacreous pigments may be chosen from white nacreous pigments such as mica coated with titanium or with bismuth oxychloride, coloured nacreous pigments such as titanium mica with iron oxides, titanium mica with, especially, ferric blue or chromium oxide, titanium mica with an organic pigment of the abovementioned type and also nacreous pigments based on bismuth oxychloride. They may represent from 0,1 to 20% and better still from 0.1% to 15% of the total weight of the composition.

Advantageously, the composition according to the invention may comprise a particulate phase present in a content ranging from 5% to 70% by weight, preferably ranging from 10% to 60% by weight and preferentially ranging from 15% to 40% by weight, relative to the total weight of the composition.

The particulate phase may comprise a particulate material chosen from the fillers, pigments and nacres as described above. The composition according to the invention may be manufactured by the known processes generally used in cosmetics or dermatology.

### *Forms of the composition*

The composition of the invention may be self-supporting and may be in the form of a stick or a dish. According to any one aspect, it is in the form of a transparent or transluscent rigid gel and more especially in the form of a transparent or transluscent stick, the liquid fatty phase forming the continuous phase. The composition could be anhydrous.

The contents of dextrin ester of formula (I) or (II) are chosen according to the desired hardness of the gel or stick and according to the particular intended application.

The hardness may be measured by the "cheese wire" method, which consists in cutting an 8.1 mm tube of lipstick and measuring the hardness at 20°C, using a DFGHS 2 tensile testing machine from the company Indelco-Chatillon pulling at a speed of 100 mm/minute. It is expressed as the shear force (expressed in grams) required to cut a stick under these conditions.

According to this method, the hardness of a composition in stick form according to the invention ranges from 30 to 150 g, preferably from 30 to 120 and for example from 30 to 50 g.

This hardness is such that the composition is self-supporting and can disintegrate readily to form a satisfactory deposit on the skin and the lips. In addition, with this hardness, the composition of the invention in cast form, especially cast as a stick, shows good impact strength.

According to the invention, the composition in stick form has the behaviour of a deformable and flexible elastic solid, giving on application noteworthy elastic softness. The prior art compositions in stick form do not have this property of elasticity and flexibility.

When these sticks or tubes are coloured and in particular pigmented, they give on application a glossy deposit of uniform colour that does not migrate in the wrinkles and fine lines of the skin, in particular surrounding the lips, but also the eyes.

According to any one embodiment, the composition is in the form of a care, cleansing, makeup-removing or makeup composition for the skin, semi-mucous membranes, mucous membranes and/or the integuments.

It may be in the form of a paste, a solid or a cream. It may be an oil-in-water or water-in-oil emulsion, a solid or soft anhydrous gel, or in the form of a free or compacted powder, and even in two-phase form. It is preferably in anhydrous form. The term "anhydrous composition" means a composition to which water is not added during formulation, and or which comprises a fatty outer or continuous phase that represents up to 98% by weight and preferably up to 99.5% by weight of the composition.

According to any one preferred embodiment, the composition is in the form of a makeup composition such as a foundation, a makeup rouge, an eye-shadow, a lipstick, a mascara or an eyeliner; a care composition such as a lipcare base, a care cream (day cream, night cream, anti-wrinkle cream or moisturizing cream), or a makeup-removing cream or emulsion; an antisun composition or self-tanning composition; a haircare composition such as a care cream for the hair, the eyelashes and the eyebrows.

### *Uses*

A subject of the invention is also the use of a dextrin ester with a degree of substitution of less than 2 on the basis of one repeating unit, for example one glucose unit,, as described above, in a composition containing a continuous liquid fatty phase, the said fatty phase containing an oil or a mixture of oils that has solubility parameters δd, δp and δh satisfying the following conditions:

$$[4(\delta d-\delta d_e)^2 + (\delta p-\delta p_e)^2 + (\delta h-\delta h_e)^2]^{1/2} \leq 10$$

$\delta d_e$, $\delta p_e$ and $\delta h_e$ being the solubility parameters of the at least one dextrin ester, to structure the said composition and/or to limit the exudation of the composition, and/or to increase the gloss of the composition, and/or to limit the migration of the deposit of the composition, and/or to give the composition transparency.

A subject of the invention is also the use of a dextrin ester of formula (I) or (II) as described above, to increase the sheen of the composition and/or to improve the stability of the composition and/or to give the composition transparency; and/or to structure the said fatty phase of the composition.

A subject of the present invention is also, when the liquid fatty phase of the composition also contains pigments and/or fillers, the use of a dextrin ester with a degree of substitution of the dextrin ester of less than 2 on the basis of one repeating unit, for example one glucose unit,, to improve the uniformity of the deposit of the composition, in particular its colour uniformity.

A subject of the invention is also the use of at least one acid ester of dextrin with a degree of substitution of less than 2 on the basis of one repeating unit, for example one glucose unit, and at least one fatty acid ester of dextrin with a degree of substitution of greater than 2 on the basis of one repeating unit, for example one glucose unit, in a cosmetic composition containing a continuous liquid fatty phase, to thicken or gel the composition and/or to increase the sheen of the composition and/or to limit the exudation of the composition, and/or to limit the migration of the composition, and/or to give the composition transparency and/or to obtain a stable composition and/or to obtain a deposit that is comfortable and/or uniform on keratin materials and/or to obtain a composition that applies easily to keratin materials.

A subject of the invention is also the use of a composition as defined above to obtain a deposit, in particular makeup, which is uniform and/or comfortable on keratin materials.

A subject of the invention is also the use of a sufficient amount of a mixture containing at least one fatty acid ester of dextrin with a degree of substitution of less than 2 on the basis of one repeating unit, for example one glucose unit, and at least one fatty acid ester of dextrin with a degree of substitution of greater than 2 on the basis of one repeating unit, for example one glucose unit,, in a composition containing a liquid fatty phase and pigments, to improve the uniformity of the deposit of the composition, in particular its uniformity of colour and/or to obtain a stable composition and/or to obtain a composition that applies easily to keratin materials.

A subject of the invention is also the use of a dextrin ester with a degree of substitution of less than 2 on the basis of one repeating unit, for example one glucose unit,, as described above, in a wax-free anhydrous composition containing a liquid fatty phase, to gel or thicken the said composition and/or to limit the exudation of the composition, and/or to increase the gloss of the composition, and/or to limit the migration of the deposit of the composition, and/or to give the composition transparency.

A subject of the present invention is also, when the liquid fatty phase of the wax-free anhydrous composition also contains pigments and/or fillers, the use of a dextrin ester with a degree of substitution of the dextrin ester of less than 2 on the basis of one repeating unit, for example one glucose unit, to improve the uniformity of the deposit of the composition, in particular its colour uniformity.

The invention is illustrated in greater detail in the examples that follow. The amounts are given as percentages by mass.

<u>Example 1:</u>

10% polyethylene wax
5% dextrin palmitate Rheopearl TL (degree of substitution 1.5 to 1.7)
85% castor oil

It is noted that this composition is in the form of a stick that does not exude at 45°C for 2 months.

<u>Comparative Example 2:</u>

10% polyethylene wax
90% castor oil

This composition is in the form of a stick that exudes when it is placed under accelerated ageing conditions at 45°C for 2 months.

Example 3 : Jar of gloss

| | |
|---|---|
| Polydecene | 21 |
| Octyldodecanol | 30 |
| Tridecyl trimellitate | 33.5 |
| Dextrin palmitate (Rhéopearl® TL) | 7.5 |
| Dextrin palmitate (Rhéopearl® KL) | 7.5 |
| Preserving agents | qs 100 |

All the ingredients are mixed together with stirring at 90°C until a uniform mixture is obtained. The mixture is poured into dishes and left to cool at room temperature.

This gloss has a transparent, shiny structure, and is easy to spread. It does not exude at 45°C for 2 months.

Example 4 :

15% of dextrin palmitate Rheopearl KL (degree of substitution of 2.1 to 2.3)
15% of dextrin palmitate Rheopearl TL (degree of substitution 1.5 to 1.7)
70% of caprylic/capric acid triglyceride

It is noted that this composition is in the form of a stick that does not exude at 45°C for 2 months.

Comparative Example 5 :

30% dextrin palmitate Rheopearl® KL (degree of substitution 2.1 to 2.3)
70% caprylic/capric acid triglyceride

This composition is in the form of a stick that exudes when it is placed under accelerated ageing conditions in an oven at 45°C for 3 months.

Example 6 :

10% of Nikkol, dextrin palmitate, sold by the company Nikko Chemicals (totally esterified)
5% Rheopearl TL
85% caprylic/capric acid triglyceride oil

This composition is in the form of a stick that does not exude at 45°C for 2 months.

Comparative Example 7 :

10% Nikkol, dextrin palmitate, sold by the company Nikko Chemicals (totally esterified)
90% caprylic/capric acid triglyceride oil

This composition is in the form of a stick that exudes, under accelerated ageing conditions at 45°C for 2 months.

Example 8:

An anhydrous foundation having the composition below was prepared:

- Dextrin palmitate (Rheopearl® TL) 5.3
- Dextrin palmitate (Rheopearl® KL) 5.3
- Isododecane qs 100
- Isohexadecane 15
- Isononyl isononanoate 13.3
- Microcrystalline wax 3.2
- Polymethyl methacrylate 15
- Kaolin 3
- Talc 3
- Nylon-12 powder 8.5 Hydrophobic coated iron oxides 1.8
- Hydrophobic coated titanium dioxide 6.7

The composition is prepared by mixing the microcrystalline wax, the isononyl isononanoate and the dextrin palmitates at 85°C; next, without heating, the premilled mixture of pigments and of isohexadecane is added. The isododecane is then added at room temperature, followed by the fillers.

A foundation that is stable at 45°C for two months is obtained. This foundation has a fondant texture when applied to the skin and forms a thin, non-sticky film. The makeup deposited on the skin shows good colour uniformity.

Example 9:

An anhydrous foundation having the composition below was prepared:

- Dextrin palmitate (Rheopearl® TLC) 4.8
- Dextrin palmitate (Rheopearl®KL) 4.8
- Isododecane qs 100
- Isohexadecane 15
- Isononyl isononanoate 16.2
- Microcrystalline wax 3.2
- Polymethyl methacrylate 7.3
- Fumed silica 2.3
- Lauroyllysine 5
- Polyurethane-silica powder sold under the name Plastic Powder D-400 by the company Toshiki) 8
- Polymethylsilsesquioxane microbeads (Tospearl from Toshiba) 5.2
- Hydrophobic coated iron oxides 2.1
- Hydrophobic coated titanium dioxide 6.3

The foundation shows good stability at 45°C for 2 months. It applies easily to the face, giving a fondant effect, and forms a thin, non-sticky, uniform film.

## Claims

**1.** Composition containing a continuous liquid fatty phase gelled or thickened with a sufficient amount of at least one fatty acid ester of dextrin with a degree of substitution of less than 2 on the basis of one repeating unit, for example one glucose unit,, the said fatty phase containing an oil or a mixture of oils that has solubility parameters δd, δp and δh satisfying the following conditions:

$$[4(\delta d-\delta d_e)^2 + (\delta p-\delta p_e)^2 + (\delta h-\delta h_e)^2 ]^{1/2} \leq 10$$

$\delta d_e$, $\delta p_e$ and $\delta h_e$ being the solubility parameters of the dextrin ester, the liquid fatty phase and the dextrin ester forming a physiologically acceptable medium.

**2.** Composition containing a continuous liquid fatty phase gelled or thickened with a sufficient amount of a mixture

containing at least one fatty acid ester of dextrin with a degree of substitution of less than 2 on the basis of one repeating unit, for example one glucose unit, and at least one fatty acid ester of dextrin with a degree of substitution of greater than 2 on the basis of one repeating unit, for example one glucose unit,, the liquid fatty phase and the dextrin esters forming a physiologically acceptable medium.

**3.** Wax-free anhydrous composition containing a continuous liquid fatty phase thickened or gelled with a sufficient amount of at least one fatty acid ester of dextrin with a degree of substitution of less than 2 on the basis of one repeating unit, for example one glucose unit to thicken or gel said liquid fatty phase, the liquid fatty phase and the dextrin ester forming a physiologically acceptable medium.

**4.** Composition according to the preceding claims, **characterized in that** the fatty acid ester of dextrin with a degree of substitution of less than 2 on the basis of a repeating unit, corresponds to formula (I) :

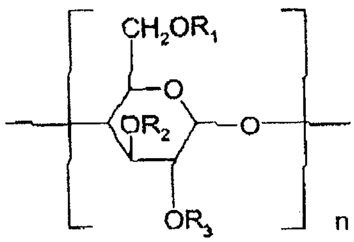

(I)

in which:

- the radicals $R_1$, $R_2$ and $R_3$, which may be identical or different, are chosen from hydrogen or an acyl group (R-CO-) in which the radical R is a linear or branched, saturated or unsaturated hydrocarbon-based group containing 6 to 50, especially 8 to 30, or even 12 to 22 and better still 12 to 18 carbon atoms, with the proviso that at least one of the said radicals $R_1$, $R_2$ or $R_3$ is other than hydrogen,
- n is an integer between 3 and 150, especially 10 and 100, and preferably 15-40.

**5.** Composition according to the preceding claims, **characterized in that** the fatty acid ester of dextrin with a degree of substitution of less than 2 corresponds to formula (II):

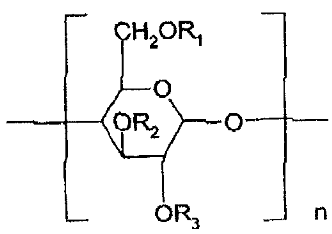

(II)

in which:

- the radicals $R_1$, $R_2$ and $R_3$ represent hydrogen or an acyl group (R-CO-) in which the radical R is a linear or branched, saturated or unsaturated hydrocarbon-based group containing 6 to 50, especially 8 to 30, or even 12 to 22 and better still 12 to 18 carbon atoms, with the proviso that at least one of the said radicals $R_1$, $R_2$ or $R_3$ is other than hydrogen,
- n is an integer between 3 and 150, especially 10 and 100, and preferably 15-40.

**6.** Composition according to any one of the preceding claims, **characterized in that** the fatty acid ester of dextrin with a degree of substitution of greater than 2 corresponds to formula (III) :

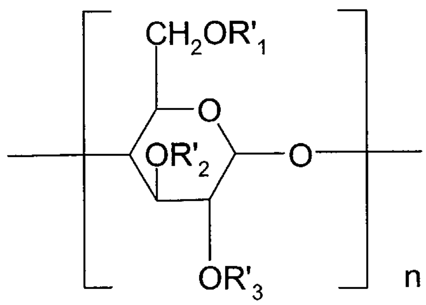

(III)

in which:

- the radicals R'1, R'2 and R'3, which may be identical or different, are chosen from hydrogen or an acyl group (R'-CO-) in which the radical R' is a linear or branched, saturated or unsaturated hydrocarbon-based group containing 6 to 50, especially 8 to 30, or even 12 - 22 and better still 12 - 18 carbon atoms, with the proviso that at least one of the said radicals R'1, R'2 or R'3 is other than hydrogen,
- n is an integer between 3 and 150, especially between 10 and 100 and preferably 15 - 40.

**7.** Composition according to any one of Claims 4 to 6, **characterized in that** R-CO- or R'-CO- is chosen from caprylyl, caproyl, lauroyl, myristyl, palmityl, stearyl, eicosanyl, docosanoyl, isovaleryl, 2-ethylbutyryl, ethylmethylacetyl, isoheptanyl, 2-ethylhexanyl, isononanyl, isodecanyl, isotridecanyl, isomyristyl, isopalmityl, isostearyl, isohexanyl, decenyl, dodecenyl, tetradecenyl, myristyl, hexadecenoyl, palmitoleyl, oleyl, elaidyl, eicosenyl, sorbyl, linoleyl, linolenyl, punicyl, arachidonyl and stearoyl radicals, and mixtures thereof.

**8.** Composition according to any one of Claims 4 to 7, **characterized in that** the radical R or R' is linear.

**9.** Composition according to any one of Claims 4 to 7, **characterized in that** R-CO- or R'-CO- is the palmitic radical.

**10.** Composition according to any one of the preceding claims, **characterized in that** the degree of substitution of the dextrin ester with a degree of substitution less than 2 is less than 1.9, preferably less than 1.8 and more preferably between 1.5 and 1.7.

**11.** Composition according to any one of the Claims 4 and 7, **characterized in that** n ranges from 25 to 35 and preferably from 27 to 33, and better still is equal to 30.

**12.** Composition according to any one of the preceding claims, **characterized in that** the weight-average molecular weight of the dextrin ester is between 10 000 and 30 000 and preferably between 15 000 and 20 000.

**13.** Composition according to any one of the preceding claims, **characterized in that** the dextrin ester with a degree of substitution of greater than 2 has a degree of substitution of between 2.1 and 2.3.

**14.** Composition according to any one of the preceding claims, **characterized in that** the fatty acid ester of dextrin with a degree of substitution of less than 2 on the basis of one repeating unit, for example one glucose unit, may be present in the composition according to the invention in an amount ranging from 1% to 50%, preferably from 4 % to 30 %, preferably from 4% to 25%, preferably from 5% to 25%, preferably from 4 % to 25 % and preferably from 10% to 25% by weight, relative to the total weight of the composition.

**15.** Composition according to any one of the preceding claims, **characterized in that** the mixture of dextrin esters is present in an amount of less than or equal to 50%, preferably from 1 % to 50 %, preferably from 5% to 40% and preferentially from 5% to 30% by weight, and most preferably from 9 % to 25 %, relative to the total weight of the composition.

**16.** Composition according to any one of the preceding claims, **characterized in that** the fatty acid ester of dextrin with a degree of substitution of less than 2 and the fatty acid ester of dextrin with a degree of substitution of greater than 2 are in relative mass proportions ranging from 1/10 to 3/1 and preferably from 1/2 to 3/2, preferably from 0.8 to 1.2, preferably from 0.9 to 1.1, and particularly from 1/2 to 1/1.

**17.** Composition according to claim 1, **characterized in that** the oil is chosen from fatty acid triglycerides of 4 to 22 carbon atoms, hydroxylated triglycerides, synthetic alcohols containing from 12 to 26 carbon atoms, and C4-22 fatty acid esters, in particular castor oil.

**18.** Composition according to any one of the preceding claims, **characterized in that** the liquid fatty phase comprises a volatile organic oil.

**19.** Composition according to the preceding claim, **characterized in that** the volatile organic oil is chosen from octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane, isododecane, isodecane and isohexadecane, and mixtures thereof.

**20.** Composition according either of Claims 18 or 19, **characterized in that** the volatile organic oil represents from 0.1% to 80%, preferably from 1% to 60% and better still from 5% to 50% of the total weight of the composition.

**21.** Composition according to any one of claims 2 or 3, **characterized in that** the liquid fatty phase comprises a non-volatile oil.

**22.** Composition according to Claim 21, **characterized in that** the non-volatile oil is present in a content ranging from 0.1% to 80% by weight, preferably ranging from 1% to 60% by weight and preferentially ranging from 5% to 50% by weight, relative to the total weight of the composition.

**23.** Composition to any one of the preceding claims, **characterized in that** the liquid fatty phase comprises from 5% to 95% by weight of oil(s), preferably from 20% to 75% by weight and preferentially from 30% to 70% by weight, relative to the total weight of the composition.

**24.** Composition according to claim 1, **characterized in that**

$$[4(\delta d-\delta d_e)^2 + (\delta p-\delta p_e)^2 + (\delta h-\delta h_e)^2]^{1/2} \leq 8.$$

**25.** Composition according to claim 1, **characterized in that**

$$[4(\delta d-\delta d_e)^2 + (\delta p-\delta p_e)^2 + (\delta h-\delta h_e)^2]^{1/2} \leq 5.$$

**26.** Composition according to any one of Claims 15 to 17, **characterized in that** the values of the Hansen solubility parameters for the fatty acid ester of dextrin with a degree of substitution of less than 2 are especially such that $17 \leq \delta d_e \leq 19$, $1 \leq \delta p_e \leq 2$ and $9 \leq \delta h_e \leq 11$.

**27.** Composition according to claim 1 or 3, **characterized in that** it contains an additional non-waxy gelling or thickening system such as:

- N-lauryl L-glutamate α,γ-di-N-butylamide,
- monodibenzylidene sorbitol,
- 1,2- and 1,3-cyclohexane derivatives bearing an amide function,
- palmitates of dextrins and of fatty acid with a degree of substitution of greater than 2 relative to one glucose unit,
- mixtures thereof.

**28.** Composition according to claim 2, **characterized in that** it contains an additional non-waxy gelling or thickening system such as:

- N-lauryl L-glutamate α,γ-di-N-butylamide,
- monodibenzylidene sorbitol,
- 1,2- and 1,3-cyclohexane derivatives bearing an amide function,
- mixtures thereof.

**29.** Composition according to any one of the preceding claims, **characterized in that** it also contains at least one

dyestuff chosen from lipophilic dyes, hydrophilic dyes, pigments and nacres, and mixtures thereof.

**30.** Composition according to Claim 31, **characterized in that** the dyestuff is present in a proportion of from 0.01% to 50% and preferably from 5% to 25% relative to the total weight of the composition.

**31.** Composition according to any one of the preceding claims, **characterized in that** it is in solid or semi-solid form.

**32.** Composition according to any one of the preceding claims, **characterized in that** it is in the form of a rigid gel, especially a stick.

**33.** Composition according to any one of the preceding claims, **characterized in that** it is in the form of a mascara, an eyeliner, a foundation, a lipstick, a blusher, a deodorant product or makeup-removing product, a makeup product for the body, an eye-shadow, a makeup rouge, a concealer product, a shampoo, a conditioner, an antisun composition or a care product for the face and the body.

**34.** Composition according to any one of the preceding claims, **characterized in that** it is in the form of a tube of lipstick, a lip balm, a lip gloss or a composition to be applied over a film of lipstick.

**35.** Composition according to any one of the preceding claims, **characterized in that** it is free of waxes.

**36.** Composition according to any one of the preceding claims, **characterized in that** it is anhydrous.

**37.** Use of a dextrin ester with a degree of substitution of less than 2 on the basis of one repeating unit, for example one glucose unit, in a composition containing a continuous liquid fatty phase, the said fatty phase containing an oil or a mixture of oils that has solubility parameters δd, δp and δh satisfying the following conditions:

$$[4(\delta d-\delta d_e)^2 + (\delta p-\delta p_e)^2 + (\delta h-\delta h_e)^2]^{1/2} \leq 10$$

$\delta d_e$, $\delta p_e$ and $\delta h_e$ being the solubility parameters of the dextrin ester, to increase the gloss of the composition and/or to improve the stability of the composition.

**38.** Use of a dextrin ester with a degree of substitution of less than 2 on the basis of one repeating unit, for example one glucose unit, in a composition containing a continuous liquid fatty phase and pigments, to improve the uniformity of the deposit of the composition, in particular its colour uniformity.

**39.** Use of a dextrin ester with a degree of substitution of less than 2 on the basis of one repeating unit, for example one glucose unit, in a wax-free anhydrous composition containing a liquid fatty phase, to increase the gloss of the composition and/or to improve the stability of the composition and/or to give the composition transparency and/or to gel or thicken the said fatty phase of the composition.

**40.** Use of a dextrin ester with a degree of substitution of less than 2 on the basis of one repeating unit, for example one glucose unit, in a wax-free anhydrous composition containing a liquid fatty phase and pigments, to increase the uniformity of the deposit of the composition, in particular its colour uniformity.

**41.** Use of a sufficient amount of a mixture containing at least one fatty acid ester of dextrin with a degree of substitution of less than 2 on the basis of one repeating unit, for example one glucose unit, and at least one fatty acid ester of dextrin with a degree of substitution of greater than 2 on the basis of one repeating unit, for example one glucose unit, in a composition containing a liquid fatty phase, to increase the sheen of the composition and/or to improve the stability of the composition, and/or to limit the exudation of the composition, and/or to give the composition transparency, and/or to gel or thicken the said fatty phase of the composition and/or to obtain a stable composition and/or to obtain a composition that applies easily to keratin materials.

**42.** Use of a sufficient amount of a mixture containing at least one fatty acid ester of dextrin with a degree of substitution of less than 2 on the basis of one repeating unit, for example one glucose unit, and at least one fatty acid ester of dextrin with a degree of substitution of greater than 2 on the basis of one repeating unit, for example one glucose unit,, in a composition containing a liquid fatty phase and pigments, to improve the uniformity of the deposit of the composition, in particular its uniformity of colour and/or to obtain a stable composition and/or to obtain a composition

that applies easily to keratin materials.

European Patent Office

# EUROPEAN SEARCH REPORT

Application Number
EP 03 29 1845

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DATABASE WPI<br>Week 200277<br>Derwent Publications Ltd., London, GB;<br>AN 2002-709440<br>XP002240607<br>& JP 2002 193747 A (KOSE CO.)<br>* abstract *<br>--- | 1-42 | A61K7/027 |
| A | DATABASE CA [Online]<br>CHEMICAL ABSTRACTS SERVICE, COLUMBUS, OHIO, US;<br>Database accession no. 1997:53719<br>XP002240604<br>* abstract * | 1-42 | |
| A | & JP 08 283303 A (CHIBA SEIFUN CO.)<br>29 October 1996 (1996-10-29)<br>* abstract *<br>--- | 1-42 | |
| A | DATABASE CA [Online]<br>CHEMICAL ABSTRACTS SERVICE, COLUMBUS, OHIO, US;<br>Database accession no. 2002:513054<br>XP002240605<br>* abstract * | 1-42 | TECHNICAL FIELDS SEARCHED (Int.Cl.7)<br>A61K |
| A | & JP 2002 193741 A (KOSEI CO.)<br>10 July 2002 (2002-07-10)<br>* abstract *<br>--- | 1-42 | |
| A | DATABASE CA [Online]<br>CHEMICAL ABSTRACTS SERVICE, COLUMBUS, OHIO, US;<br>Database accession no. 2001:159405<br>XP002240606<br>* abstract * | 1-42 | |
| A | & JP 2001 058922 A (KOSEI CO.)<br>6 March 2001 (2001-03-06)<br>* abstract *<br>----- | 1-42 | |

The present search report has been drawn up for all claims

| Place of search | Date of completion of the search | Examiner |
|---|---|---|
| THE HAGUE | 27 November 2003 | Willekens, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 29 1845

This annex lists the patent family members relating to the patent documents cited in the above-mentioned European search report.
*The members are as contained in the European Patent Office EDP file on*
The European Patent Office is in no way liable for these particulars which are merely given for the purpose of information.

27-11-2003

| Patent document cited in search report | Publication date | Patent family member(s) | Publication date |
|---|---|---|---|
| JP 2002193747 A | 10-07-2002 | NONE | |
| JP 8283303 A | 29-10-1996 | JP 3236926 B2 | 10-12-2001 |
| JP 2002193741 A | 10-07-2002 | NONE | |
| JP 2001058922 A | 06-03-2001 | NONE | |

EPO FORM P0459

For more details about this annex : see Official Journal of the European Patent Office, No. 12/82